# EUROPEAN PATENT APPLICATION

(11) **EP 4 282 421 A1**
(43) Date of publication of application: **29.11.2023**
(21) Application number: 22742726.7
(22) Date of filing: 25.01.2022
(51) Int. Cl.: A61K 31/7088, A61P 25/08, C12N 15/113

(54) **PREVENTIVE OR THERAPEUTIC AGENT FOR BENIGN ADULT FAMILIAL MYOCLONIC EPILEPSY**

(30) Priority: 25.01.2021 JP 2021009903
(71) Applicant: RIKEN, Wako-shi, Saitama 351-0198 (JP)
(72) Inventor: INOUE, Haruhisa, Wako-shi, Saitama 351-0198 (JP); SUGA, Mika, Wako-shi, Saitama 351-0198 (JP); KONDO, Takayuki, Wako-shi, Saitama 351-0198 (JP); TAKAHASHI, Ryosuke, Kyoto-shi, Kyoto 606-8501 (JP); IKEDA, Akio, Kyoto-shi, Kyoto 606-8501 (JP)
(74) Representative: J A Kemp LLP
(86) International application number: PCT/JP2022/002705
(87) International publication number: WO 2022/158608

(57) **Abstract**

The preventive or therapeutic agent for benign adult familial myoclonus epilepsy (BAFME) of the present invention contains an antisense oligonucleotide containing at least one of the base sequences of SEQ ID NOs: 1 to 4. Among the antisense oligonucleotides, oligonucleotide consisting of at least one of the base sequences of SEQ ID NOs: 1 to 4 may contain at least one RNase H inactive type nucleotide analog. The RNase H inactive type nucleotide analog may be β-D-oxy-L-LNA, β-D-ENA, R type cEt, 2'-OMe-nucleotide analog, and/or MOE-nucleotide analog.

## Description

### [Technical Field]

The present invention relates to preventive or therapeutic agents for benign adult familial myoclonus epilepsy, specifically to preventive or therapeutic agents for benign adult familial myoclonus epilepsy containing antisense oligonucleotides.

### [Background Art]

Benign adult familial myoclonus epilepsy (hereinafter to be referred to as "BAFME") is one type of progressive myoclonus epilepsy, in which the main symptoms are myoclonus (involuntary movements including sudden convulsion of a part of the body) and epilepsy seizures (systemic convulsive seizure, loss of consciousness).

The first symptoms of progressive myoclonus epilepsy are systemic convulsive seizures and myoclonus of the whole body or parts of the body. When the symptom is myoclonus alone, consciousness is preserved and the disease may be unnoticed. As the symptoms progress, dizziness when walking, symptoms of forgetfulness, dementia, mental symptoms, and the like may appear. It causes irreversible abnormalities in specific areas of the brain.

BAFME has conventionally been considered "benign" and "nonprogressive" because it develops after adulthood and initially has mild symptoms and progresses slowly. However, recent studies have revealed that it is progressive, and symptoms are particularly exacerbated and activities of daily living (ADL) decline in old age. Epidemiologically, many cases are found in Japan and China, and 60 families have been reported in Japan. The onset frequency is 1 in 35,000.

BAFME is also referred to as familial cortical myoclonic tremor associated with epilepsy (FCMTE) or familial adult myoclonic epilepsy (FAME)). BAFME is classified into type 1 to type 7 according to age of onset, disease progression, linkage analysis, and the like. In the following, it is referred to as BAFME1, BAFME2,..., BAFME7.

Ishiura et al. (Non Patent Literature 1) identified using data from Japanese patients, and Cen et al. (Non Patent Literature 2) confirmed using data from Chinese patients that BAFME1, BAFME6, and BAFME7 are caused by elongation of 5-base repetitive sequence in noncoding regions within introns of genes SAMD12, TNRC6A, and PAPGEF2, respectively, on different autosomes.

The findings about the position of the causative gene on the chromosome, the name of the causative gene, the number of patients (the number of families in parentheses), the repetitive sequence registered in the reference sequence Hg19 of the human genome, and the constitution of the elongated repetitive sequence in the genome of Japanese patients for BAFME1, BAFME6, and BAFME7 are summarized in the following Table 1. The subscripted numbers in the reference sequence and the elongated repetitive sequence in Table 1 mean the number of repeats of the repetitive sequence in parentheses. The subscript "exp" in the extended repetitive sequence in Table 1 indicates that the 5-base repetitive sequence in parentheses is repeated multiple times and elongated. The number of repeats varies depending on patient and gene locus. For example, repeat numbers of 440 to 3680 were observed in BAFME1. In addition, it is known that patients with a higher number of repeats develop epilepsy and myoclonus at lower age.

**[Table 1]**

| disease | chromosome | gene | | number of patients (number of families) | reference sequence (Hg19) | constitution of elongated repetitive sequence |
|---|---|---|---|---|---|---|
| BAFME1 | 8q24.11-24.12 | SAMD12 | repeat structure 1 | 82 (48) | (TTTTA)₇TTA(TTTTA)₁₃ | (TTTTA)ₑₓₚ(TTTCA) ₑₓₚ |
| | | | repeat structure 2 | 3(1) | | (TTTTA)ₑₓₚ(TTTCA)ₑₓₚ(TTTTA)ₑₓₚ |
| BAFME6 | 16p21 . 1 | TNRC6A | | 5 (1) | (TTTTA)₁₈ | (TTTTA)₂₂(TTTCA)ₑₓₚ(TTTTA)ₑₓₚ |
| BAFME7 | 4q32.1 | RAPGE F2 | | 1 (1) | (TTTTA)₅TATTA(TTTTA)₁₂ | (TTTTA)ₑₓₚ(TTTCA)ₑₓₚ(TTTTA)ₑₓₚ |

In the sequences, exp means that 5-base repetitive sequence in the parentheses is repeatedly elongated multiple times.

In BAFME1, RNA aggregates are detected in cortical neurons and Purkinje cells by fluorescence in situ hybridization (hereinafter referred to as "FISH") using a probe for the repetitive sequence TTTCA (Non Patent Literature 1).

### [Citation List]

### [Non Patent Literature]

[NPL 1]
   Ishiura, H. et al., Nature Genetics, 50: 581-590. (2018)
[NPL 2]
   Cen, Z. et al., Brain, 141: 2280-2288 (2018)

### [Summary of Invention]

### [Technical Problem]

A causal therapy of BAFME has not been established yet, and treatments by symptomatic therapy with continued use of medications to relieve or eliminate epileptic seizures and myoclonus have been performed.

The present invention aims to provide a preventive or therapeutic agent for BAFME as a causal therapy of BAFME.

### [Solution to Problem]

The present inventors have conducted intensive studies in an attempt to achieve the above-mentioned objects and found that a disease model system using iPS cells derived from BAFME type 1 disease patients is suitable for screening ASO with high removing activity of RNA aggregate , and completed the present invention by using the assay system.

The present invention provides a preventive or therapeutic agent for BAFME. The preventive or therapeutic agent of the present invention contains ASO containing at least one of the base sequences of SEQ ID NOs: 1 to 4.

In the preventive or therapeutic agent of the present invention, an oligonucleotide consisting of at least one of the base sequences of SEQ ID NOs: 1 to 4 may contain at least one RNase H inactive type nucleotide analog.

In the preventive or therapeutic agent of the present invention, the aforementioned RNase H inactive type nucleotide analog may be at least one kind of nucleotide analog selected from a nucleotide analog in which the 2'-position of ribose is modified and a nucleotide analog modified by bridging between the 2'-position and the 4'-position of ribose.

In the preventive or therapeutic agent of the present invention, the aforementioned nucleotide analog in which the 2'-position of ribose is modified may be at least one kind of nucleotide analog selected from the group consisting of a 2'-OMe-nucleotide analog and an MOE-nucleotide analog.

In the preventive or therapeutic agent of the present invention, the aforementioned nucleotide analog modified by bridging between the 2'-position and the 4'-position of ribose may be at least one kind of nucleotide analog selected from the group consisting of β-D-oxy-L-LNA, β-D-ENA, and R type cEt.

In the preventive or therapeutic agent of the present invention, at least two adjacent nucleotides of the oligonucleotide consisting of a base sequence of any of SEQ ID NOs: 1 to 4 in the aforementioned ASO may be linked by a modified internucleoside bond.

In the preventive or therapeutic agent of the present invention, all adjacent nucleotides of the oligonucleotide consisting of a base sequence of any of SEQ ID NOs: 1 to 4 in the aforementioned ASO may be linked by a modified internucleoside bond.

In the preventive or therapeutic agent of the present invention, the aforementioned modified internucleoside bond may be at least one kind of bond selected from the group consisting of a phosphorothioate bond, a phosphorodithioate bond, and a boranophosphate bond.

In the preventive or therapeutic agent of the present invention, the oligonucleotide consisting of a base sequence of any of SEQ ID NOs: 1 to 4 in the aforementioned ASO may be of RNase H active type.

In the preventive or therapeutic agent of the present invention, the 1st to 5th nucleotides from the 5'-terminal and the 16th to 20th nucleotides from the 5'-terminal of the oligonucleotide consisting of a base sequence of any of SEQ ID NOs: 1 to 4 in the aforementioned RNase H active type oligonucleotide may be at least two kinds of RNase H inactive type nucleotide analogs.

In the preventive or therapeutic agent of the present invention, the 1st to 5th nucleotides from the 5'-terminal and the 16th to 20th nucleotides from the 5'-terminal of the oligonucleotide consisting of a base sequence of any of SEQ ID NOs: 1 to 4 in the aforementioned RNase H active type oligonucleotide may all be the same kind of RNase H inactive type nucleotide analogs.

In the preventive or therapeutic agent of the present invention, the 1st to 5th nucleotides from the 5'-terminal and the 16th to 20th nucleotides from the 5'-terminal of the oligonucleotide consisting of a base sequence of any of SEQ ID NOs: 1 to 4 in the aforementioned RNase H active type oligonucleotide may be RNase H inactive type nucleotide analogs, and the 6th to 15th nucleotides from the 5'-terminal of the oligonucleotide therein may be RNase H active type nucleotides.

In the preventive or therapeutic agent of the present invention, at least one of the aforementioned RNase H inactive type nucleotide analogs in the aforementioned ASO containing the RNase H active type oligonucleotide may be β-D-ENA.

In the preventive or therapeutic agent of the present invention, the aforementioned RNase H inactive type nucleotide analogs may all be β-D-ENA and the aforementioned modified internucleoside bonds may all be phosphorothioate bonds in the aforementioned ASO containing the RNase H active type oligonucleotide.

In the preventive or therapeutic agent of the present invention, the aforementioned RNase H inactive type nucleotide analogs may all be β-D-ENA, the aforementioned RNase H active type nucleotides may all be deoxyribonucleotides, and the internucleoside bonds of the aforementioned RNase H active type oligonucleotide may all be phosphorothioate bonds, in the aforementioned ASO containing the RNase H active type oligonucleotide.

In the preventive or therapeutic agent of the present invention, the aforementioned ASO containing an oligonucleotide consisting of the base sequence of SEQ ID NO: 1, 2, or 3 among the aforementioned ASOs may be of RNase H inactive type.

In the preventive or therapeutic agent of the present invention, all nucleotides including thymine of the oligonucleotide consisting of the base sequence of SEQ ID NO: 1 or 2 may be nucleotide analogs modified by bridging between the 2'-position and the 4'-position of ribose, nucleotides containing the 5th, 10th, 15th, and 20th adenines from the 5'-terminal of the aforementioned oligonucleotide consisting of the base sequence of SEQ ID NO: 1 or 2 may be nucleotide analogs modified by bridging between the 2'-position and the 4'-position of ribose, and other nucleotides may be all nucleotide analogs in which the 2'-position of ribose is modified.

In the preventive or therapeutic agent of the present invention, at least one of the aforementioned nucleotide analogs modified by bridging between the 2'-position and the 4'-position of ribose in the oligonucleotide consisting of the base sequence of SEQ ID NO: 1 or 2 may be β-D-ENA, and at least one of the aforementioned nucleotide analogs in which the 2'-position of ribose is modified may be a 2'-OMe-nucleotide analog.

In the preventive or therapeutic agent of the present invention, all the aforementioned nucleotide analogs modified by bridging between the 2'-position and the 4'-position of ribose in the oligonucleotide consisting of the base sequence of SEQ ID NO: 1 or 2 may be β-D-ENA, and all the aforementioned nucleotide analogs in which the 2'-position of ribose is modified may be 2'-OMe-nucleotide analogs.

In the preventive or therapeutic agent of the present invention, all nucleotides including thymine of the oligonucleotide consisting of the base sequence of SEQ ID NO: 3 may be nucleotide analogs modified by bridging between the 2'-position and the 4'-position of ribose, and all other nucleotides may be nucleotide analogs in which the 2'-position of ribose is modified.

In the preventive or therapeutic agent of the present invention, at least one of the aforementioned nucleotide analogs modified by bridging between the 2'-position and the 4'-position of ribose may be β-D-ENA, and at least one of the aforementioned nucleotide analogs in which the 2'-position of ribose is modified may be a 2'-OMe-nucleotide analog.

In the preventive or therapeutic agent of the present invention, all the aforementioned nucleotide analogs modified by bridging between the 2'-position and the 4'-position of ribose may be β-D-ENA, and the aforementioned nucleotide analogs in which the 2'-position of ribose is modified may be 2'-OMe-nucleotide analogs.

In the preventive or therapeutic agent of the present invention, all the aforementioned nucleotide analogs modified by bridging between the 2'-position and the 4'-position of ribose may be β-D-ENA, the aforementioned nucleotide analog in which the 2'-position of ribose is modified may be a 2'-OMe-nucleotide analog, and all internucleoside bonds of the aforementioned RNase H inactive type oligonucleotide may be phosphorothioate bonds.

The present invention provides an assay system for a preventive or therapeutic agent for BAFME type 1 disease, containing undifferentiated cells of iPS cells established from a human affected with BAFME type 1 disease.

### [Brief Description of Drawings]

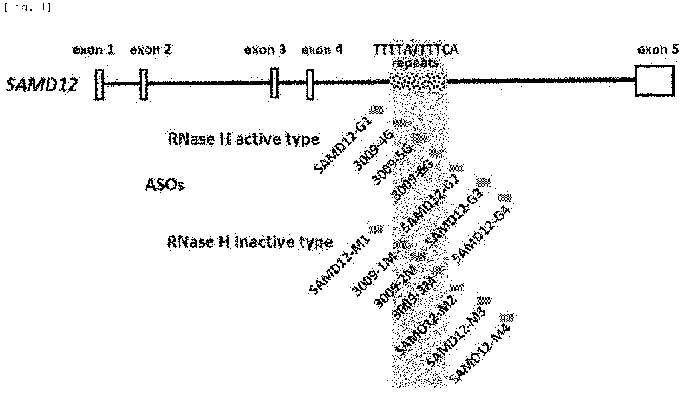

Fig. 1 is a schematic diagram showing the relative positional relationship of the target sites of the ASOs of the Examples of the present application in the pre-mRNA of the SAMD12 gene, and the classification of the aforementioned ASOs into RNase H active type or inactive type. (See Table 3 for details of the aforementioned ASOs.)

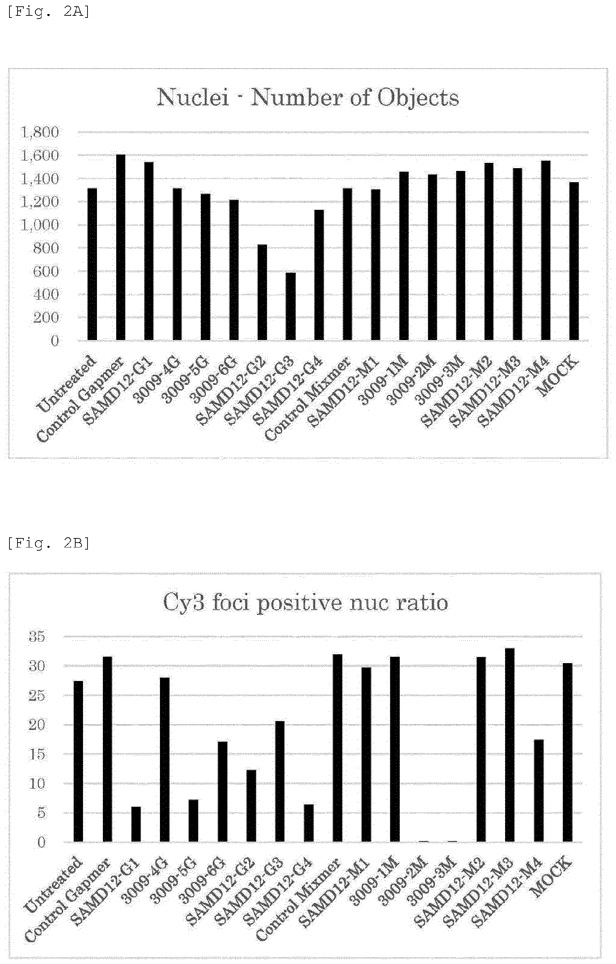

Fig. 2A is a graph showing the results of the experiment in which the ASOs of the Examples of the present application and negative control ASO (Control Gapmer) or the like instead of the ASOs of the Examples of the present application were introduced into undifferentiated cells of iPS cells (HPS3905) derived from BAFME patient by lipofection. The height of the bar graph indicates the percentage of the number of iPS cell nuclei 48 hr after lipofection.
Fig. 2B is a graph showing the results of the experiment in which the ASOs of the Examples of the present application and negative control ASO (Control Gapmer) or the like instead of the ASOs of the Examples of the present application were introduced into undifferentiated cells of iPS cells (HPS3905) derived from BAFME patient by lipofection. The height of the bar graph indicates the percentage of iPS cell nuclei in which RNA aggregates were detected with the Cy3 probe among the iPS cell nuclei 48 hr after lipofection.

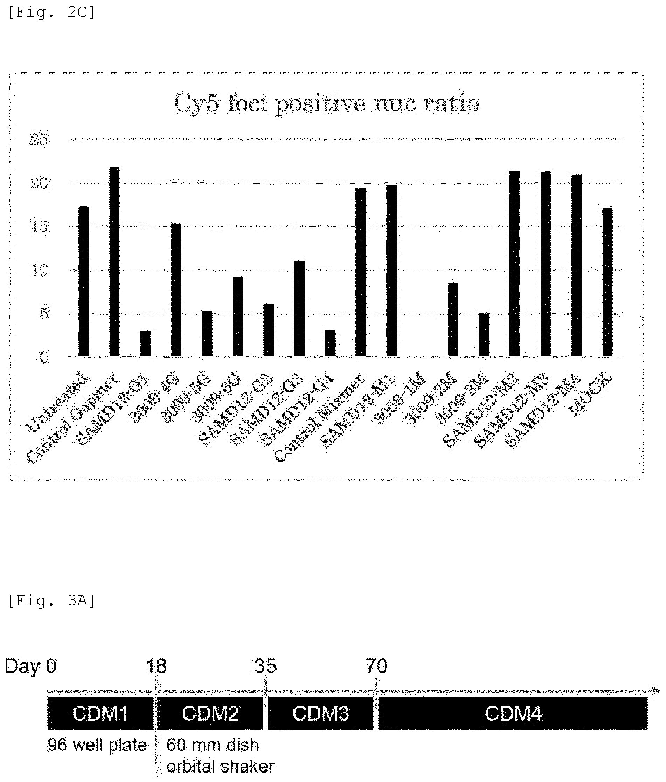

Fig. 2C is a graph showing the results of the experiment in which the ASOs of the Examples of the present application and negative control ASO (Control Gapmer) or the like instead of the ASOs of the Examples of the present application were introduced into undifferentiated cells of iPS cells (HPS3905) derived from BAFME patients by lipofection. The height of the bar graph indicates the percentage of iPS cell nuclei in which RNA aggregates were detected with the Cy5 probe among the iPS cell nuclei 48 hr after lipofection.
Fig. 3A is a schematic diagram showing an overview of the procedure for producing a cerebral organoid in the Examples of the present application.

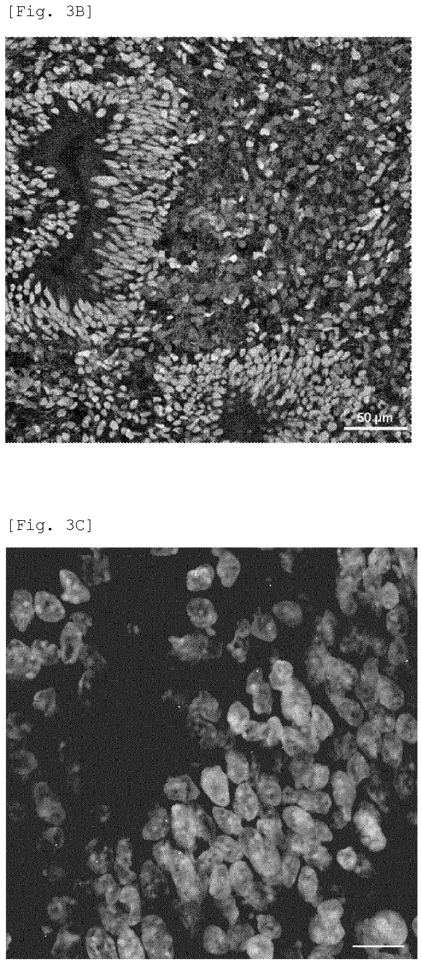

Fig. 3B shows multiple immunostaining fluorescence microscopic images of cerebral organoid sections obtained by culturing iPS cell line HPS3905 derived from BAFME patients for 70 days. The scale bar is 50 µm.
Fig. 3C shows fluorescence microscopic images of FISH using Cy3 probe and DAPI counterstaining of cerebral organoid sections obtained by culturing iPS cell line HPS3905 derived from BAFME patients for 70 days. The scale bar is 10 µm.

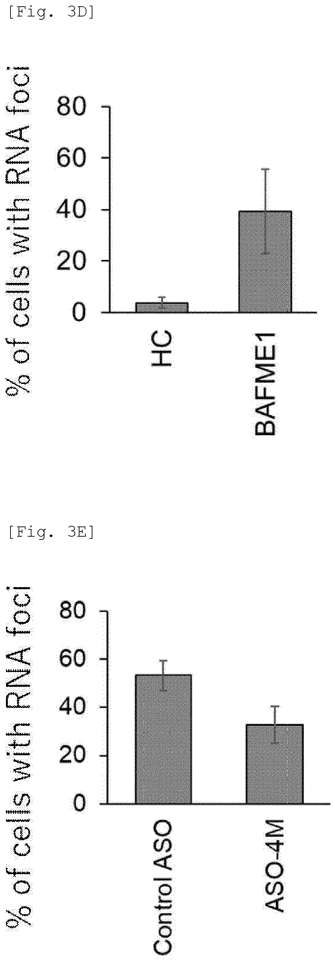

Fig. 3D is a graph showing the percentage of cell nuclei in which RNA aggregates were detected with the Cy3 probe in the cell nuclei of cerebral organoid sections obtained by culturing iPS cell line HPS3905 (BAFME1) derived from BAFME patients and iPS cell line 201B7 (HC) derived from healthy subject for 70 days.
Fig. 3E is a graph showing the effect of the ASO of the present Example (ASO-4M, same as 3009-2M) or negative control ASO (Control ASO, same as Control Mixmer) on RNA aggregate formation in cerebral organoids prepared from iPS cells derived from BAFME patients. It shows the percentage of cell nuclei in which RNA aggregates were detected with the Cy3 probe in the cell nuclei of sections obtained by culturing iPS cell line HPS3905 derived from BAFME patients for 5 months to give cerebral organoids, administering thereto ASO-4M or Control ASO for 1 week by lipofection, and fixing.

### [Description of Embodiments]

One embodiment of the present invention is a preventive or therapeutic agent for BAFME containing ASO having at least one of the base sequences of SEQ ID NOs: 1 to 4.

The base sequences of the oligonucleotides described in the present specification are listed as SEQ ID NOs: 1-7 in the Sequence Listing attached to the present specification. The correspondence between the aforementioned SEQ ID NOs and base sequences is shown in Table 2.

**[Table 2]**

| SEQ ID NO: | base sequence |
|---|---|
| 1 | TGA AAT GAA ATG AAA TGA AA |
| 2 | TGA AAT GAA ATA AAA TAA AA |
| 3 | GTA GTT GAC ACT TAG TAG GT |
| 4 | AGG ATA AGG ATA GAA GGC TT |
| 5 | GCA GGA AAA TTG CTT GAA AC |
| 6 | CAC GGT GGC TCA TGC CTG TA |
| 7 | TAA AAT AAA ATA AAA TAA AA |

In Table 2, SEQ ID NOs: 1 to 7 are the base sequences of ASOs administered to iPS cells in the Examples of the present specification. In the Sequence Listing attached to the present specification, the sequences of SEQ ID NOs: 1-7 are listed as DNA. However, the sugar moieties of the nucleosides constituting the oligonucleotides contained in the preventive or therapeutic agent for BAFME of the present invention are not necessarily limited to deoxyribose, and may be the sugar moieties of modified nucleotides described below.

Among the ASOs administered to iPS cells in the Examples of the present specification, 3009-1M and 3009-4G are ASOs complementary to RNA corresponding to the TTTTA repetitive sequence in the fourth intron of the SAMD12 gene. 3009-2M and 3009-5G are ASOs complementary to RNA corresponding to the TTTCA repetitive sequence in the fourth intron of the SAMD12 gene. 3009-3M and 3009-6G are ASOs complementary to both the RNA corresponding to the TTTTA repetitive sequence and the RNA corresponding to the TTTCA repetitive sequence in the fourth intron of the SAMD12 gene. SAMD12-1M and SAMD12-1G are ASOs complementary to RNA corresponding to a non-repetitive sequence on the 5'-side (upstream) from the TTTTA repetitive sequence initiation site in the fourth intron of the SAMD12 gene. SAMD12-2M and SAMD12-2G, and SAMD12-3 M and SAMD12-3G are ASOs complementary to RNAs corresponding to different non-repetitive sequences on the 3'-side (downstream) of the TTTTA repetitive sequence end site in the fourth intron of the SAMD12 gene. ASOs containing the letter "M" in the names of these ASOs are RNase H inactive type ASOs. ASOs containing the letter "G" in the names of these ASOs are RNase H active type ASOs. The names, types, structures, and target sites in the fourth intron of the SAMD12 gene of the ASOs used in the Examples in the present specification are shown in the following Table 3.

**[Table 3]**

| ASO | type | base sequence | target site | SEQ ID NO: |
|---|---|---|---|---|
| 3009-2M | Mixmer type | TGA AAT GAA ATG AAA TGA AA | TTTCA repetitive sequence | 1 |
| 3009-5G | Gapmer type | TGA AAT GAA ATG AAA TGA AA | | |
| 3009-3M | Mixmer type | TGA AAT GAA ATA AAA TAA AA | TTTTA/TTTCA repetitive sequence | 2 |
| 3009-6G | Gapmer type | TGA AAT GAA ATA AAA TAA AA | | |
| SAMD12-M4 | Mixmer type | GTA GTT GAC ACT TAG TAG GT | between 355-375 bases 3' downstream from TTTTA repetitive sequence end site | 3 |
| SAMD12-G4 | Gapmer type | GTA GTT GAC ACT TAG TAG GT | | |
| SAMD12-M1 | Mixmer type | AGG ATA AGG ATA GAA GGC TT | between 71-51 bases 5' upstream from TTTTA repetitive sequence initiation site | 4 |
| SAMD12-G1 | Gapmer type | AGG ATA AGG ATA GAA GGC TT | | |
| SAMD12-M2 | Mixmer type | GCA GGA AAA TTG CTT GAA AC | between 80-100 bases 3' downstream from TTTTA repetitive sequence end site | 5 |
| SAMD12-G2 | Gapmer type | GCA GGA AAA TTG CTT GAA AC | | |
| SAMD12-M3 | Mixmer type | CAC GGT GGC TCA TGC CTG TA | between 258-278 bases 3' downstream from TTTTA repetitive sequence end site | 6 |
| SAMD12-G3 | Gapmer type | CAC GGT GGC TCA TGC CTG TA | | |
| 3009-1M | Mixmer type | TAA AAT AAA ATA AAA TAA AA | TTTTA repetitive sequence | 7 |
| 3009-4G | Gapmer type | TAA AAT AAA ATA AAA TAA AA | | |
| Control Mixmer | Mixmer type | CAC GCG AGA CAC GCG AGA CGA | | 8 |
| Control Gapme | Gapmer type | CCT ATA GGA CTA TCC AGG AA | | 9 |

In all ASOs, phosphodiester moieties of internucleoside linkage are substituted with phosphorothioate groups.

Underlined nucleotides are substituted with β-D-ENA.

In the Gapmer type, nucleotides not underlined are deoxyribonucleotides.

In the Mixmer type, nucleotides not underlined are substituted with 2'-OMe-nucleotide analogs.

In all ASOs in Table 3, all adjacent nucleotides are linked by phosphorothioate bonds, and bases "A", "G", "C", and "T" are nucleotides containing adenine, guanine, cytosine, and thymine, respectively, and underlined nucleotides are β-D-ENA-nucleic acids (hereinafter to be referred to as "ENA"). In RNase H active type ASOs, nucleotides not underlined are deoxyribonucleotides. In RNase H inactive type ASOs, nucleotides not underlined are 2'-OMe-nucleotide analogs. The nucleotide analogs that can be used in the ASO of the present invention are described in detail below.

One embodiment of the present invention is a preventive or therapeutic agent for BAFME containing ASO, wherein an oligonucleotide consisting of at least one of the base sequences of SEQ ID NOs: 1 to 4 contains at least one RNase H inactive type nucleotide analog.

That a specific nucleotide analog is RNase H inactive type means that, when a hetero double-stranded nucleic acid formed by pairing a single-stranded nucleic acid composed of the aforementioned nucleotide analog and a single-stranded RNA complementary to the single-stranded nucleic acid is reacted with RNase H, the phosphodiester bond of the aforementioned RNA is not hydrolyzed. Conversely, that a specific nucleotide analog is RNase H active type means that, when a hetero double-stranded nucleic acid formed by pairing a single-stranded nucleic acid composed of the aforementioned nucleotide analog and a single-stranded RNA complementary to the single-stranded nucleic acid is reacted with RNase H, the phosphodiester bond of the aforementioned RNA is hydrolyzed.

In the present specification, that a specific oligonucleotide "contains at least one RNase H inactive type nucleotide analog" means that at least one nucleotide of the oligonucleotide is the nucleotide analog described below. In the present specification, that a specific nucleotide "is RNase H inactive type nucleotide" means that the aforementioned nucleotide has the same base moiety and a sugar moiety which is the sugar moiety of the RNase H inactive type nucleotide analogue described below.

One embodiment of the present invention is a preventive or therapeutic agent for BAFME containing ASO, wherein the aforementioned RNase H inactive type nucleotide analog is at least one kind of nucleotide analog selected from the group consisting of a nucleotide analog in which the 2'-position of ribose is modified and a nucleotide analog modified by bridging between the 2'-position and the 4'-position of ribose.

The nucleotide analog modified at the 2'-position of ribose to be contained in the ASO in the aforementioned embodiment includes, but is not limited to, 2'-F nucleotide analog, 2'-OMe-nucleotide analog, MOE-nucleotide analog, 2'-(3-hydroxy)propylnucleotide analog, and 2'-AP nucleotide analog.

The aforementioned nucleotide analogs modified at the 2'-position of ribose is represented by, for example, the following chemical formula (1) wherein substituent X to be bonded to the 2'-position of ribose is selected from a fluoro group, a methoxy group, an O-methoxyethyl group, a 2'-(3-hydroxy)propyl group, and a 2'-(3-amino)propyl group, Z and Z* are each independently selected from an internucleotide linkage, R, a terminal group, and a protecting group, B is selected from natural or non-natural nucleotide base moieties (nucleic acid bases), and R is selected from hydrogen, a hydroxyl group, a C₁₋₄ alkyl group, and a C₁₋₄ alkoxy group.

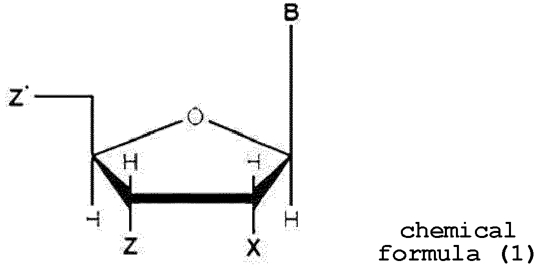

In chemical formula (1), a nucleotide analog in which substituent X to be bonded to the 2'-position of ribose is a fluoro group is a 2'-F nucleotide analog, a nucleotide analog in which substituent X to be bonded to the 2'-position of ribose is a methoxy group is a 2'-OMe-nucleotide analog, a nucleotide analog in which substituent X to be bonded to the 2'-position of ribose is an O-methoxyethyl group is a MOE-nucleotide analog, a nucleotide analog in which substituent X to be bonded to the 2'-position of ribose is a 2'-(3-hydroxy)propyl group is a 2'-(3-hydroxy)propylnucleotide analog, and a nucleotide analog in which substituent X to be bonded to the 2'-position of ribose is a 2'-(3-amino)propyl group is a 2'-AP nucleotide analog.

One embodiment of the present invention is a preventive or therapeutic agent for BAFME containing ASO, wherein the aforementioned nucleotide analog in which the 2'-position of ribose is modified is at least one kind of nucleotide analog selected from the group consisting of a 2'-OMe-nucleotide analog and an MOE-nucleotide analog.

One embodiment of the present invention is a preventive or therapeutic agent for BAFME containing ASO, wherein the aforementioned nucleotide analog in which the 2'-position of ribose is modified is a 2'-OMe-nucleotide analog.

The nucleotide analog modified by bridging between the 2'-position and the 4'-position of ribose to be contained in the oligonucleotide of the aforementioned embodiment includes, but is not limited to, oxy-LNA, thio-LNA, amino-LNA, 5'-methyl-LNA, ENA, cEt, and cMOE.

The aforementioned nucleotide analog modified by bridging between the 2'-position and the 4'-position of ribose is also referred to as locked nucleic acid or LNA, because the conformation of the 2'-position and the 4'-position is fixed (locked). Alternatively, it is also referred to as BNA or bicyclic nucleic acid because it is crosslinked (bridged). The aforementioned nucleotide analog modified by bridging between the 2'-position and the 4'-position of ribose includes oxy-LNA, thio-LNA, amino-LNA, 5'-methyl-LNA, ENA, cEt, and cMOE. Among these, oxy-LNA, thio-LNA, amino-LNA, 5'-methyl-LNA, and ENA have stereoisomers of β-D-configuration and α-L-configuration, and cEt and cMOE have R and S stereoisomers having the carbon atom bridging between the 2'-position and the 4'-position of the aforementioned ribose as the asymmetric center.

The aforementioned nucleotide analog modified by bridging between the 2'-position and the 4'-position of ribose has, for example, a structure of the following chemical formulas (2) and (3) wherein a divalent atomic group "-Y-" to be bonded to the 2'-position of ribose is selected from "-O-", "-S-", "-CH₂-S-", "-N(H)-", "-N(R^{a})-", "-CH₂-N(H) -", and "-CH₂-N(R^{a})-", Z and Z* are each independently selected from an internucleotide linkage, R^{b}, a terminal group, and a protecting group, B is selected from natural or non-natural nucleotide base moieties (nucleic acid bases), R^{a} is independently selected from hydrogen and C₁₋₄ alkyl, and R^{b} is selected from hydrogen, a hydroxyl group, a C₁₋₄ alkyl group, and a C₁₋₄ alkoxy group.

The oxy-LNA, thio-LNA, and amino-LNA with β-D-configuration are represented by the formula (2), and oxy-LNA, thio-LNA, and amino-LNA with α-L-configuration are represented by the formula (3). In the following chemical formulas (2) and (3), a nucleotide analog in which a divalent atomic group "-Y-" to be bonded to the 2'-position of ribose is "-O-" is oxy-LNA, a nucleotide analog in which a divalent atomic group "-Y-" to be bonded to the 2'-position of ribose is "-S-" or "-CH₂-S-" is thio-LNA, and a nucleotide analog in which a divalent atomic group "-Y-" to be bonded to the 2'-position of ribose is selected from "-N(H)-", "-N(R^{a})-", "-CH₂-N(H)-", and "-CH₂-N(R^{a})-" (wherein R^{a} is selected from C₁₋₄ alkyl) is amino-LNA.

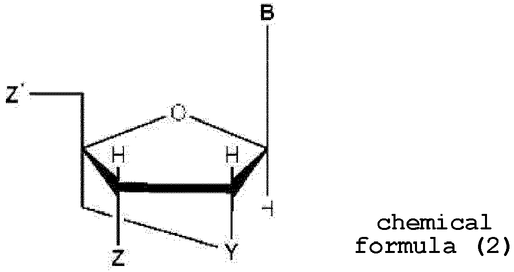

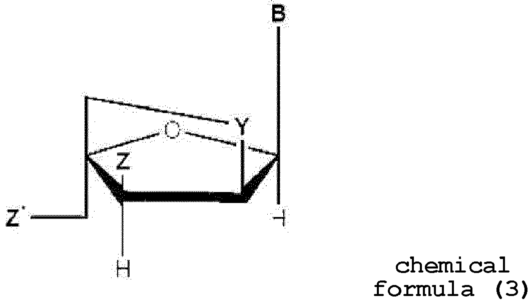

The 5'-methyl-LNA with β-D-configuration is represented by the following chemical formula (4), and 5'-methyl-LNA with α-L-configuration is represented by the following chemical formula (5).

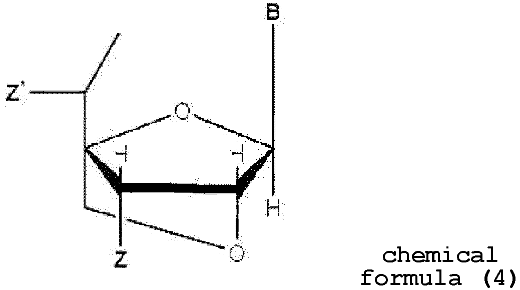

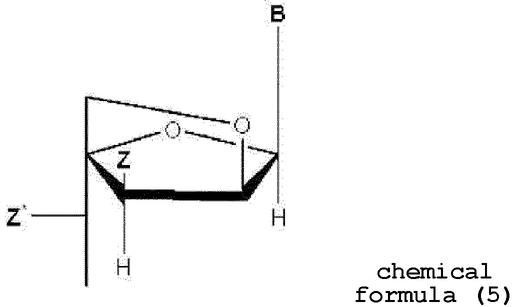

The ENA with β-D-configuration is represented by the following chemical formula (6), and ENA with α-L-configuration is represented by the following chemical formula (7).

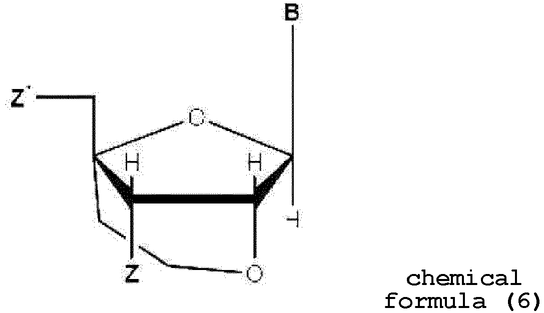

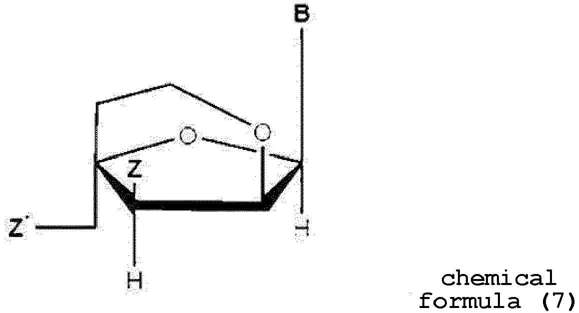

R-type cEt and cMOE are represented by the following formula (8), and S-type cEt and cMOE are represented by the following formula (9). In the structures of the chemical formulas (8) and (9), W is selected from a methyl group and a methoxymethyl group, Z and Z* are independently selected from an internucleotide linkage, R (R is selected from hydrogen, hydroxyl group, C₁₋₄ alkyl group, and C₁₋₄ alkoxy group), a terminal group, and a protecting group, and B is selected from natural or non-natural nucleotide base moieties (nucleic acid bases).

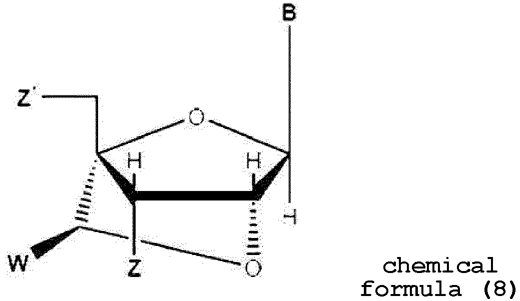

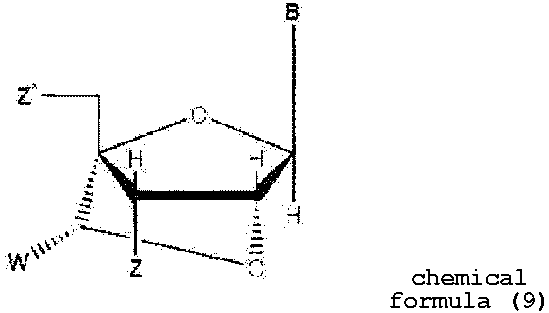

In the chemical formulas (8) and (9), a nucleotide analogue in which W is a methyl group is cEt, and a nucleotide analogue in which W is a methoxymethyl group is cMOE.

One embodiment of the present invention is a preventive or therapeutic agent for BAFME containing ASO, wherein the aforementioned nucleotide analog modified by bridging between the 2'-position and the 4'-position of ribose is at least one kind of nucleotide analog selected from the group consisting of β-D-oxy-L-LNA, β-D-ENA, and R type cEt.

One embodiment of the present invention is a preventive or therapeutic agent for BAFME containing ASO, wherein the aforementioned nucleotide analog modified by bridging between the 2'-position and the 4'-position of ribose is β-D-ENA.

Among the nucleotide analogues, ENA is a nucleotide analogue that is highly resistant to nuclease degradation and thermodynamically stable, and ASOs that have incorporated ENA have high affinity for complementary RNA strands (Morita, K. et al., Bioorganic & Medicinal Chemistry, 11: 2211-2226.(2003)).

One embodiment of the present invention is a preventive or therapeutic agent for BAFME containing ASO, in which at least two adjacent nucleotides of the oligonucleotide consisting of a base sequence of any of SEQ ID NOs: 1 to 4 are linked by a modified internucleoside bond.

The nucleoside monomers of the oligonucleotides contained in the preventive or therapeutic agent of the present invention are linked via an internucleoside bond. Each monomer is appropriately linked to the 3' adjacent monomer via an internucleoside bond. The terminal 5' monomer of the oligonucleotide may or may not contain a 5' terminal group or an atomic group for conjugation, but does not contain an internucleoside bond.

The term "internucleoside bond" refers to a group that can link two nucleosides with a covalent bond to form a dinucleotide. Natural nucleotides are linked by a phosphodiester bond. In contrast, a modified internucleoside bond includes, but is not limited to, phosphorothioate bond, phosphorodithioate bond, and boranophosphate bond. Generally, oligonucleotides containing modified internucleoside bonds have higher membrane permeability and higher resistance to intracellular and extracellular nucleases than oligonucleotides linked only by phosphodiester bonds. On the other hand, a hetero double strand nucleic acid formed by hybridizing an oligonucleotide containing a modified internucleoside bond with RNA in a cell is degraded by RNase H in the same way as hetero double strand nucleic acids formed by hybridizing an oligonucleotide linked only by phosphodiester bonds with RNA in a cell. In addition, modified internucleoside bond have stereoisomers. For example, phosphorothioate bonds have stereoisomers about the sulfur atom. The oligonucleotide contained in the preventive or therapeutic agent of the present invention may be synthesized such that modified internucleoside bonds of a specific stereoisomer are used for specific internucleotide linkage of the oligonucleotide, or may be synthesized such that a part or all of the internucleotide linkages is/are racemate(s).

In the present specification, when a particular nucleotide is described to be an RNase H active type nucleotide, an RNase H inactive type nucleotide, or a nucleotide having deoxyribose as a sugar moiety, the linkage between the nucleotide and the adjacent nucleotide may be a modified internucleoside bond. For example, the sequences of SEQ ID NOs: 1-7 are listed as DNA in the Sequence Listing attached to the present specification. However, at least two adjacent nucleotides in the oligonucleotide contained in the preventive or therapeutic agent for BAFME of this embodiment may be linked by a modified internucleoside bond.

One embodiment of the present invention is a preventive or therapeutic agent for BAFME containing ASO, wherein all adjacent nucleotides in the oligonucleotide consisting of a base sequence of any of SEQ ID NOs: 1 to 4 are linked by a modified internucleoside bond.

One embodiment of the present invention is a preventive or therapeutic agent for BAFME containing ASO, wherein the aforementioned modified internucleoside bond is at least one kind of group selected from the group consisting of a phosphorothioate bond, a phosphorodithioate bond, and a boranophosphate bond.

One embodiment of the present invention is a preventive or therapeutic agent for BAFME containing ASO, wherein the aforementioned modified internucleoside bond is a phosphorothioate bond.

One embodiment of the present invention is a preventive or therapeutic agent for BAFME wherein the oligonucleotide consisting of a base sequence of any of SEQ ID NOs: 1 to 4 contains RNase H active type ASO.

An RNase H active type ASO refers to an ASO in which, when a hetero double strand is formed with the target RNA, the target RNA are cleaved by RNase H activity between nucleosides and the target RNA is degraded. In contrast, an RNase H inactive type ASO refers to an ASO in which, when a hetero double strand is formed with the target RNA, internucleoside linkages of the target RNA are not cleaved by RNase H activity and the target RNA is not degraded. RNase H active type ASO has a structure in which several RNase H active type nucleotides are consecutive. The structure of RNase H active type ASO includes a structure consisting only of RNase H active type nucleotides, and a structure in which RNase H inactive type nucleotide is linked to the 5'-terminal side and/or the 3'-terminal side of several consecutive RNase H active type nucleotides. Among the structures of RNase H active type ASO, a structure in which RNase H inactive type nucleotide is linked to the 5'-terminal side and the 3'-terminal side of several consecutive RNase H active type nucleotides is particularly referred to as a gapmer type structure.

In the gapmer type, several consecutive RNase H inactive type nucleotides on the 5'-terminal side and the 3'-terminal side of the oligonucleotide complementary to the ASO target RNA are called "wing region". The RNase H active type nucleotides in the central portion sandwiched between the wing regions are referred to as the "gap region". The oligonucleotides in the wing region have high binding affinity to complementary RNA and thus form stable double strands with the target RNA. Since oligonucleotide in the wing region has a high binding affinity to complementary RNA, a stable double strand with the target RNA is formed. Since RNase H active type nucleotides are consecutive in the oligonucleotide in the gap region, the hetero double strand formed by the pairing of the oligonucleotide in the gap region with the target RNA is degraded by RNase H. Therefore, it is preferable to employ a gapmer structure for ASO that affords efficacy when the double strand with RNA is degraded. The length of the wing region can be easily determined by a person skilled in the art according to the number of full-length nucleotides of the ASO, and can be 3, 4, 5, 6, 7, 8 or more nucleotides. The length of the gap region can also be easily determined by a person skilled in the art according to the number of full-length nucleotides of the ASO. According to Monia, B.P. et al. (J. Biological. Chem., 268: 14514 (1993)), the degradation activity by RNase H against a hetero double strand nucleic acid composed of a gapmer ASO in which a gap region consisting of at least 5 consecutive RNase H active type nucleotides is sandwiched between wing regions of RNase H inactive type nucleotides, and an RNA complementary to the ASO exceeded 75%, but the degradation activity by RNase H against a hetero double strand nucleic acid composed of a gapmer ASO in which a gap region consisting of not more than 4 consecutive RNase H active type nucleotides is sandwiched between wing regions of RNase H inactive type nucleotides, and an RNA complementary to the ASO was less than 20%. The length of the gap region can be at least 5 nucleotides.

In the present invention, whether a specific type of nucleotide analog is of RNase H active type or RNase H inactive type can be determined, for example, by the following procedure. An oligonucleotide with a gapmer structure in which a gap region with 10 consecutive nucleotide analogs of the specific type is sandwiched between known RNase H inactive type nucleotides, for example, wing regions consisting of five 2'-OMe-nucleotide analogs is generated. A hetero double strand complex is prepared by pairing 300 picomoles of an oligonucleotide with the gapmer structure with 1500 picomoles of RNA to be paired with the oligonucleotide. The hetero double strand complex and 3.0 units of Escherichia coli RNase H are added to a reaction solution for RNase H, for example, a reaction solution consisting of 50 mM Tris-HCl (pH 8.0), 75 mM KCl, 3 mM MgCl₂, and 10 mM dithiothreitol, to make a total volume of 150 µL, reacted at 37°C for 60 min, and the reaction product is analyzed by an agarose gel electrophoresis method.

One embodiment of the present invention is a preventive or therapeutic agent for BAFME, containing RNase H active type ASO in which the 1st to 5th nucleotides from the 5'-terminal and the 16th to 20th nucleotides from the 5'-terminal of the oligonucleotide consisting of a base sequence of any of SEQ ID NOs: 1 to 4 are RNase H inactive type nucleotide analogs.

Since the base sequences of SEQ ID NOs: 1 to 4 all have 20 full-length nucleotides, in this embodiment, the number of nucleotides in the wing region on the 5'-terminal side and the 3'-terminal side is 5 for each. When the ASO contained in the preventive or therapeutic agent for BAFME of the present invention consists of the base sequence of any of SEQ ID NOs: 1 to 4, the number of nucleotides of the ASO is the same as the number of bases of the base sequence of any of the aforementioned SEQ ID NOs: 1 to 4. However, when the number of nucleotides of ASO contained in the preventive or therapeutic agent for BAFME of the present invention is greater than the number of bases of the base sequence of any of the aforementioned SEQ ID NOs: 1 to 4, the "5'-terminal" of "the 1st to 5th nucleotides from the 5'-terminal" and "the 16th to 20th nucleotides from the 5'-terminal" in this embodiment is not the 5'-terminal of ASO but refers to the nucleotide corresponding to the 5'-terminal of the base sequence of any of the aforementioned SEQ ID NOs: 1 to 4 in the base sequence of ASO.

The "aforementioned RNase H inactive type nucleotide analog" in this embodiment is, as explained for other embodiments of the present invention, at least one kind of nucleotide analog selected from the group consisting of a nucleotide analog in which the 2'-position of ribose is modified, and a nucleotide analog modified by bridging between the 2'-position and the 4'-position of ribose. A nucleotide analog modified at the 2'-position of ribose includes, but is not limited to, 2'-F nucleotide analog, 2'-OMe-nucleotide analog, MOE-nucleotide analog, 2'-(3-hydroxy)propylnucleotide analog, and 2'-AP nucleotide analog. The nucleotide analog modified by bridging between the 2'-position and the 4'-position of ribose includes, but is not limited to, oxy-LNA, thio-LNA, amino-LNA, 5'-methyl-LNA, ENA, cEt, and cMOE.

One embodiment of the present invention is a preventive or therapeutic agent for BAFME, containing the aforementioned RNase H active type ASO, wherein the aforementioned RNase H inactive type nucleotide analog is β-D-ENA.

β-D-ENA is preferred for use in the wing region of gapmer-type structures because it exhibits high affinity for complementary strand nucleic acids and is highly resistant to nucleases.

One embodiment of the present invention is a preventive or therapeutic agent for BAFME, containing ASO wherein the oligonucleotide consisting of a base sequence of SEQ ID NO: 1, 2 or 3 is of RNase H inactive type.

RNase H inactive type ASO is a single-stranded oligonucleotide consisting only of RNase H inactive type nucleotides or a single-stranded oligonucleotide in which RNase H active type nucleotides and RNase H inactive type nucleotides are mixed, and a hetero double-stranded nucleic acid formed by pairing the single-stranded oligonucleotide with complementary RNA refers to one that is not degraded by RNase H. According to the aforementioned Monia, B.P. et al. (J. Biological. Chem., 268: 14514 (1993)), only not more than 5 or 4, for example, 5, 4, 3, or 2, RNase H active type nucleotides may be consecutive in the oligonucleotide in which RNase H active type nucleotide and RNase H inactive type nucleotide are mixed. Oligonucleotide consisting of the base sequence of SEQ ID NO: 1, 2, or 3 among the ASOs contained in the preventive or therapeutic agent for BAFME of this embodiment is either one in which only not more than 5 or 4, for example, 5, 4, 3, or 2, RNase H active type nucleotides are consecutive, one in which all RNase H active type nucleotides are sandwiched between one or more RNase H inactive type nucleotides, or one consisting only of RNase H inactive type nucleotides with no RNase H active type nucleotides.

One embodiment of the present invention is a preventive or therapeutic agent for BAFME, containing RNase H inactive type ASO, in which
all nucleotides including thymine of the oligonucleotide consisting of the base sequence of SEQ ID NO: 1 or 2 are nucleotide analogs modified by bridging between the 2'-position and the 4'-position of ribose, nucleotides containing the 5th, 10th, 15th, and 20th adenines from the 5'-terminal of the oligonucleotide consisting of the base sequence of SEQ ID NO: 1 or 2 are nucleotide analogs modified by bridging between the 2'-position and the 4'-position of ribose, and other nucleotides are all nucleotide analogs in which the 2'-position of ribose is modified.

Also in this embodiment, when the ASO contained in the preventive or therapeutic agent for BAFME of the present invention consists of the base sequence of SEQ ID NO: 1 or 2, the number of nucleotides of the ASO is the same as the number of bases of the base sequence of the aforementioned SEQ ID NO: 1 or 2. However, when the number of nucleotides of ASO contained in the preventive or therapeutic agent for BAFME of the present invention is greater than the number of bases of the base sequence of the aforementioned SEQ ID NO: 1 or 2, the "5'-terminal" of "the 5th, 10th, 15th, and 20th adenines from the 5'-terminal" in this embodiment is not the 5'-terminal of ASO but refers to the nucleotide corresponding to the 5'-terminal of the base sequence of the aforementioned SEQ ID NO: 1 or 2 in the base sequence of ASO.

The "aforementioned RNase H inactive type nucleotide analog" in this embodiment is, as explained for other embodiments of the present invention, at least one kind of nucleotide analog selected from the group consisting of a nucleotide analog in which the 2'-position of ribose is modified, and a nucleotide analog modified by bridging between the 2'-position and the 4'-position of ribose. A nucleotide analog modified at the 2'-position of ribose includes, but is not limited to, 2'-F nucleotide analog, 2'-OMe-nucleotide analog, MOE-nucleotide analog, 2'-(3-hydroxy)propylnucleotide analog, and 2'-AP nucleotide analog. The nucleotide analog modified by bridging between the 2'-position and the 4'-position of ribose includes, but is not limited to, oxy-LNA, thio-LNA, amino-LNA, 5'-methyl-LNA, ENA, cEt, and cMOE.

One embodiment of the present invention is a preventive or therapeutic agent for BAFME, containing RNase H inactive type ASO of an oligonucleotide consisting of a base sequence of SEQ ID NO: 1 or 2, wherein all the aforementioned nucleotide analogs modified by bridging between the 2'-position and the 4'-position of ribose are β-D-ENA, and the aforementioned nucleotide analog in which the 2'-position of ribose is modified is 2'-OMe-nucleotide analog.

β-D-ENA is preferred for use in RNase H inactive type ASO because it exhibits high affinity for complementary strand nucleic acids and is highly resistant to nucleases. 2'-OMe-nucleotide analog is preferred for use in RNase H inactive type ASO because it is more chemically stable than DNA and RNA and is more resistant to degradation by DNA and RNA-specific nuclease.

One embodiment of the present invention is a preventive or therapeutic agent for BAFME, containing RNase H inactive type ASO, in which
all nucleotides including cytosine and thymine of the oligonucleotide consisting of the base sequence of SEQ ID NO: 3 are nucleotide analogs modified by bridging between the 2'-position and the 4'-position of ribose, and other nucleotides are all nucleotide analogs in which the 2'-position of ribose is modified.

The "aforementioned RNase H inactive type nucleotide analog" in this embodiment is, as explained for other embodiments of the present invention, at least one kind of nucleotide analog selected from the group consisting of a nucleotide analog in which the 2'-position of ribose is modified, and a nucleotide analog modified by bridging between the 2'-position and the 4'-position of ribose. A nucleotide analog modified at the 2'-position of ribose includes, but is not limited to, 2'-F nucleotide analog, 2'-OMe-nucleotide analog, MOE-nucleotide analog, 2'-(3-hydroxy)propylnucleotide analog, and 2'-AP nucleotide analog. The nucleotide analogs modified by bridging between the 2'-position and the 4'-position of ribose include, but are not limited to, oxy-LNA, thio-LNA, amino-LNA, 5'-methyl-LNA, ENA, cEt, and cMOE.

One embodiment of the present invention is a preventive or therapeutic agent for BAFME, containing the aforementioned RNase H inactive type ASO, wherein all the aforementioned nucleotide analogs modified by bridging between the 2'-position and the 4'-position of ribose are β-D-ENA, and the aforementioned nucleotide analog in which the 2'-position of ribose is modified is 2'-OMe-nucleotide analog.

One embodiment of the present invention is a preventive or therapeutic agent for BAFME, containing a pharmaceutically acceptable carrier or diluent. As the carrier to be contained in the preventive or therapeutic agent of the present invention, those of ordinary skill in the art can select a carrier suitable for the situation. Carriers that can be selected include, but are not limited to, preservatives such as sodium benzoate, sodium hydrogen sulfite, methylparaben, propylparaben, and the like, pH adjusters such as sodium dihydrogenphosphate, anhydrous sodium monohydrogen phosphate, citric acid, sodium citrate, and the like, isotonicity agents such as glucose, sodium chloride, potassium chloride, and the like, divalent ion regulators such as calcium chloride, magnesium chloride, and the like. These carriers are not limited to use for the purpose of exhibiting a single action, but can be used for the purpose of exhibiting multiple actions. Diluents that can be contained in the preventive or therapeutic agent of the present invention include, but are not limited to, liquid, for example, phosphate buffered saline.

The aforementioned carrier may contain a carrier for cell membrane permeation and/or intranuclear delivery, and the carrier for cell membrane permeation and/or intranuclear delivery can be bonded to or associated with the oligonucleotide in the preventive or therapeutic agent of the present invention.

The aforementioned carrier for cell membrane permeation and/or intranuclear delivery may contain peptide. Peptides as carrier for cell membrane permeation include, but are not limited to, cell membrane penetrating peptides such as oligoarginines such as octaarginine and the like, basic peptides derived from Tat protein of HIV-1, basic helical peptides derived from the Antennapedia homeodomain protein of Drosophila, and the like. Peptides as the aforementioned carrier for intranuclear delivery include, but are not limited to, peptides that are nuclear localization signals that specifically bind to importin and are derived from SV40 virus large T antigen, c-myc, nucleoplasmin, and the like.

The aforementioned carrier for cell membrane permeation and/or intranuclear delivery may contain peptide mimetics. The peptide mimetics as a carrier for cell membrane permeation and/or intranuclear delivery is a synthetic compound mimicking the structure of peptide as the aforementioned carrier for cell membrane permeation and/or intranuclear delivery. It binds to or associates with the oligonucleotide in the preventive or therapeutic agent of the present invention and promotes cell membrane permeation and/or nuclear delivery. The peptide mimetics as a carrier for cell membrane permeation and/or intranuclear delivery is a compound in which the peptide as the aforementioned carrier for cell membrane permeation and/or intranuclear delivery is entirely or partially substituted with compounds other than natural peptides, such as heterocycles, cyclic compounds, unnatural amino acids, D-amino acids, and equivalents of various functional groups.

The carrier for cell membrane permeation and/or intranuclear delivery may contain cationic polymers of DEAE dextran, polyethyleneimine (PEI), and polypropylene imine (PPI).

The carrier for cell membrane permeation and/or intranuclear delivery may contain a lipid. Lipids as the carrier for cell membrane permeation and/or intranuclear delivery may contain, without limitation, phospholipids such as 1,2-dimyristoyl-sn-glycero-3-phosphatidylcholine (DMPC), 1,2-dipalmitoyl-sn-glycero-3-phosphatidylcholine (DPPC), 1,2-distearoyl-SN-glycero-3-phosphatidylcholine (DSPC), 1-palmitoyl-2-myristoyl-SN-glycero-3-phosphatidylcholine (PMPC), 1-stearoyl-2-myristoyl-SN-glycero-3-phosphatidylcholine (SMPC), soybean-derived hydrogenated lecithin (HSPC), 1-stearoyl-2-oleyl-sn-glycero-3-phosphatidylcholine (SOPC), 1-palmitoyl-2-oleyl-sn-glycero-3-phosphatidylcholine (POPC), 1,2-dioleoyl-sn-glycero-3-phosphocholine (DOPC), 1,2-dioleoyl-SN-glycero-3-phosphoglycerol (DOPG), 1,2-dioleoyl-SN-glycero-3-phospho-L-serine (DOPS), 1,2-dioloyloyl-SN-glycero-3-phosphoric acid (DOPA), 1,2-dipalmitoyl-sn-glycero-3-phosphocholine (DPPC), 1,2-dimyristoyl-sn-glycero-3-phospho-rac-(1-glycerol) (DMPG), 1,2-dioleyl-sn-glycero-3-phospho ethanolamine (DOPE), and the like, anionic lipids such as 1,5-O-dihexadecyl-N-succinyl-L-glutamic acid and the like, cholesterol, PEG-conjugated lipids such as N-(carbonylmethoxypolyethylene glycol 2000)-1,2-distearoyl-SN-glycero-3-phosphoethanolamine and the like, and pH sensitive lipids such as 1,2-dioleyl-3-dimethylammonium propane (DODAP).

The carrier for cell membrane permeation and/or intranuclear delivery may also contain a lipitoid. Lipitoids as the aforementioned carrier for cell membrane permeation and/or intranuclear delivery refers to a synthetic compound in which peptide and/or peptide mimetics as the aforementioned carrier for cell membrane permeation and/or intranuclear delivery, and lipid as the aforementioned carrier for cell membrane permeation and/or intranuclear delivery are linked by a covalent bond.

The carrier for cell membrane permeation and/or intranuclear delivery may also contain a liposome. Liposome as the aforementioned carrier for cell membrane permeation and/or intranuclear delivery refers to a lipid membrane structure dispersed in an aqueous solvent and enclosing the aqueous solvent inside. The lipid membrane of the liposome may be a single-layer or multi-layer lipid bilayer membrane or lipid multilayer membrane. Liposomes may be particulate or reticulated. Lipid components of liposomes may include, but are not limited to, lipids as the aforementioned carriers for cell membrane permeation and/or intranuclear delivery. Lipid components of liposomes may contain lipitoid as the aforementioned carrier for cell membrane permeation and/or intranuclear delivery. The liposome as the aforementioned carrier for cell membrane permeation and/or intranuclear delivery can encapsulate an oligonucleotide according to the preventive or therapeutic agent of the present invention therein.

The carrier for cell membrane permeation and/or intranuclear delivery may also contain a lipid nanoparticle (LNP). Lipid nanoparticles as the aforementioned carrier for cell membrane permeation and/or intranuclear delivery is composed of the same lipid as the lipid component of the aforementioned liposomes and the oligonucleotide in the preventive or therapeutic agent of the present invention. As the lipid nanoparticle, pH sensitive lipids such as 1,2-dioleyl-3-dimethylammonium propane (DODAP) that are positively charged in low pH environment may be used. Unlike liposomes, lipid nanoparticles have a single lipid membrane and have a core structure filled with lipid instead of enclosing an aqueous solvent inside. Lipid nanoparticles as the aforementioned carrier for cell membrane permeation and/or intranuclear delivery may contain the oligonucleotide in the preventive or therapeutic agent of the present invention in the aforementioned core structure.

In one embodiment of the present invention, the preventive or therapeutic agent for BAFME of the present invention can be delivered by at least one parenteral administration selected from the group consisting of subcutaneous administration, intravenous administration, intraarterial administration, intramuscular administration, intraperitoneal administration, intracranial administration, and intrathecal administration. The aforementioned administration may be consecutive or chronic, short term, or intermittent. The preventive or therapeutic agent of the present invention results in downregulation of SAMD12, TNRC6A, and PAPGEF2 RNA aggregate formation for at least 30 days, at least 35 days, at least 40 days, at least 45 days, at least 50 days, at least 55 days, at least 60 days, at least 65 days, at least 70 days, at least 75 days, at least 80 days, at least 85 days, at least 90 days, at least 95 days, at least 100 days, at least 105 days, at least 110 days, at least 115 days, at least 120 days, or at least 1 year after administration.

The preventive or therapeutic agent of the present invention may be delivered by oral administration such as tablet, capsule, granule, powder, syrup, or the like. These preparations are produced by well known methods using additives such as excipients (e.g., organic excipients such as sugar derivatives such as lactose, sucrose, glucose, mannitol, sorbitol; starch derivatives such as cornstarch, potato starch, α-starch, dextrin; cellulose derivatives such as crystalline cellulose; gum arabic; dextran; organic excipients such as pullulan: and inorganic excipients such as silicate derivatives such as light silicic anhydride, synthetic aluminum silicate, calcium silicate, magnesium aluminometasilicate; phosphates such as calcium hydrogen phosphate; carbonates such as calcium carbonate; sulfates such as calcium sulfate and the like), lubricants (e.g., stearic acid metal salts such as stearic acid, calcium stearate, magnesium stearate; talc; colloid silica; waxes such as veegum, spermaceti; boric acid; adipic acid; sulfates such as sodium sulfate; glycol; fumaric acid; sodium benzoate; DL leucine; fatty acid sodium salt; lauryl sulfates such as sodium lauryl sulfate, magnesium lauryl sulfate; silicic acids such as silicic anhydride, silicic acid hydrate; and the above-mentioned starch derivative), binders (e.g., hydroxypropylcellulose, hydroxypropylmethylcellulose, polyvinylpyrrolidone, macrogol, and compounds similar to the aforementioned excipients), disintegrants (e.g., cellulose derivatives such as low-substituted hydroxypropylcellulose, carboxy group methylcellulose, carboxy group methylcellulose calcium, internally crosslinked carboxy group methylcellulose sodium; chemically-modified starch and celluloses such as carboxy group methylstarch, carboxy group methylstarch sodium, crosslinked polyvinylpyrrolidone), stabilizers (p-oxybenzoic acid esters such as methylparaben, propylparaben; alcohols such as chlorobutanol, benzyl alcohol, phenylethylalcohol; benzalkonium chloride; phenols such as phenol, cresol; thimerosal; dehydroacetic acid; and sorbic acid), flavoring agents (e.g., sweetener, acidulant, flavor and the like used generally) and the like.

The active ingredient contained in the preventive or therapeutic agent of the present invention is the aforementioned oligonucleotide (when the oligonucleotide is bound or associated with a carrier for cell membrane penetration and/or intranuclear delivery, the aforementioned oligonucleotide bound or associated with the aforementioned carrier for cell membrane penetration and/or intranuclear delivery). The ratio of the active ingredient contained in the preventive or therapeutic agent of the present invention can be appropriately determined within the range where the desired effect can be afforded. It is generally 0.01 - 100 wt%, preferably 0.1 - 99.9 wt%, more preferably 0.5 - 99.5 wt%.

The dosage for administering the preventive or therapeutic agent of the present invention must be carefully adjusted in consideration of the age, body weight, and condition of the individual to be treated, administration route, dosage form, and administration method, and the exact dosage must be determined by a physician. The actual dosage is within the discretion of the physician and may vary taking into account the particular situation of the present invention to afford the desired therapeutic effect. The dose of the preventive or therapeutic agent of the present invention is not generally defined in view of the kind of the aforementioned active ingredient, and the body weight, age, symptoms and the like of the administration subject. In the case of, for example, parenteral administration, it is desirable to administer 0.001 mg/kg body weight (preferably 0.01 mg/kg body weight) as the lower limit and 100 mg/kg body weight (preferably 10 mg/kg body weight) as the upper limit per administration, and in the case of one oral administration, it is desirable to administer 0.01 mg/kg body weight (preferably, 0.1 mg/kg body weight) as the lower limit and 1000 mg/kg body weight (preferably, 100 mg/kg body weight) as the upper limit per administration, each once to several times a day, depending on the symptoms.

The subject of administration of the preventive or therapeutic agent of the present invention is BAFME patients. As the desirable therapeutic effects used in determining the selection of the active ingredient, carrier, dosage, and administration method of the preventive or therapeutic agent of the present invention, suppression and/or reduction of the progression of clinical symptoms of BAFME (myoclonus and/or epilepsy seizures) are/is most important. However, pharmacodynamic biomarkers (RNA aggregate formation of SAMD12, TNRC6A and PAPGEF2 and/or reduction and/or elimination of deregulation of RNA-binding molecules sequestered in the RNA aggregate) can also be used as surrogate endpoints prior to clinical symptoms, when determining the selection of the active ingredient, carrier, dosage and administration method of the preventive or therapeutic agent of the present invention.

In one embodiment of the assay system of the preventive or therapeutic agent of the present invention, the assay system of the present invention includes undifferentiated iPS cells or differentiated cells of any cell type established from a human suffering from BAFME and a compound to be the evaluation target. The compound to be an evaluation target contains ASO targeting RNA as a transcription product of at least one gene selected from the group consisting of SAMD12, TNRC6A, and PAPGEF2.

One embodiment of the present invention is a method for preventing or treating BAFME, including administering the preventive or therapeutic agent of the present invention. In the method for preventing or treating BAFME of the present invention, administration of the preventive or therapeutic agent of the present invention may be direct ex vivo administration of an ASO having at least one of the base sequences of SEQ ID NOs: 1 to 4, or administration of a vector containing AAV or other virus modified to produce an oligonucleotide containing at least one of the base sequences of SEQ ID NOs: 1 to 4.

In the present specification, the adnominal "about" that modifies numerical values means a numerical range of 90% or more and 110% or less of the numerical values. For example, "about 40 bases" refers to bases in a numerical range of 36 bases or more and 44 bases or less.

All documents referred to in the present specification are hereby incorporated by reference in their entirety.

The examples of the present invention described below are for illustrative purposes only and do not limit the technical scope of the present invention. The technical scope of the present invention is limited only by the description of the CLAIMS. Modifications of the present invention, such as addition, deletion, and substitution of constituent elements of the present invention, can be made without departing from the gist of the present invention.

### [Example]

### Example 1

### Screening of ASO preventive or therapeutic agent candidates for BAFME by using patient-derived iPS cells

### (1) material and method

Using peripheral blood mononuclear cells (PBMC) collected from four BAFME patients as starting cells, iPS cells were generated by reprogramming with episomal vectors encoding SOX2, KLF4, OCT4, L-MYC, LIN28, p53 carboxyl terminal dominant negative fragment, and EBNA1 (Okita, K. et al., Stem Cells, 31:458-466(2013)). Cell line authenticity was confirmed by STR analysis using PowerPlex (registered trade mark) 16 system (Promega, Madison, Wisconsin, USA). Karyotype analysis was performed by LSI Medience Corporation (Tokyo). iPS cells were cultured and maintained in StemFit (registered trade mark) medium (AK02N, Ajinomoto Co., Inc., Tokyo) explained by Nakagawa, M. et al. (Sci Rep, 4: 3594 (2014)) on a culture vessel coated with iMatrix-511 (892012, Nippi, Incorporated, Tokyo). In the following Examples, undifferentiated cells of HPS3905, in which a BAFME-iPSC strain in which RNA aggregates are detected, were used for ASO lipofection and detection of RNA aggregates by the FISH method.

In order to detect nuclear RNA aggregates, FISH using a Cy3-labeled DNA/LNA probe that targets UUUCA (hereinafter referred to as "Cy3") and a Cy5-labeled DNA/LNA probe that targets UUUUA (hereinafter referred to as "Cy5") was performed. Cells were cultured on clear-bottomed microplates (CellCarrier-96 Ultra, PerkinElmer), fixed with 4% paraformaldehyde for 30 min at room temperature, membrane permeabilized with 70% ethanol at -30°C for at least 2 hr, rehydrated with a 1×SSC solution prepared with diethyl dicarbonate (DEPC) water, and incubated in a hybridization pre-treatment buffer [2×SSC and 50% formamide prepared with DEPC water] at 37°C for 2 hr. Thereafter, the cells were incubated in a hybridization buffer [2×SSC, 50% formamide, 10% dextrin sulfate and 0.1 ng/µL of Cy3 probe and Cy5 probe (GeneDesign, Inc., Osaka)] overnight at 37°C. The cells were washed twice with the aforementioned hybridization pre-treatment buffer at 37°C for 20 min, and washed twice at room temperature for 5 min with 1×SSC solution prepared with DEPC water. The cells were further incubated with 1×SSC prepared in DEPC water at 37°C for 60 min and washed twice with 1×SSC prepared in DEPC water. The cells were stained with 1 µg/mL 4',6-diamidino-2-phenylindole (DAPI, Invitrogen, Thermo Fisher Scientific) in PBS at room temperature for 10 min and washed with PBS. Imaging was analyzed with the Opera Phenix (trademark) High Content Screening System (PerkinElmer) using a 40x water immersion objective lens.

Fig. 1 is a schematic diagram showing the relative positional relationship of the target sites of the ASOs of the Examples of the present application in the pre-mRNA of the SAMD12 gene, and the classification of the aforementioned ASOs into RNase H active type or inactive type. The names, the presence or absence of RNase H activity, structures, and target sites in the fourth intron of the SAMD12 gene of the aforementioned ASOs are shown in Table 3. Among ASOs, 3009-1M to 3009-3M that target the TTTTA/TTTCA repetitive sequence, and 3009-4G to 3009-6G target not only the SAMD12 gene, which is the causative gene of BAFME1, but also the TTTTA/TTTCA repetitive sequences of the TNRC6A gene and RAPGEF2 gene, which are the causative genes of BAFME6 and BAFME7. Since the fourth intron of the SAMD12 gene is as long as about 180,000 bases, the sequences of candidate targets for SAMD12-G1 to SAMD12-G4 and SAMD12-M1 to SAMD12-M4 were designed in the range of 1,000 bases upstream and downstream of the TTTTA/TTTCA repetitive sequence, using the Sfold software described in Ding, Y. et al. (Nucleic Acids Research, Vol. 32, Web Server issue W135-W141(2004)).

The underlined nucleotides in the structure of each ASO in Table 3 are ENAs and obtained from KNC Laboratories Co., Ltd. (Kobe). Nucleotides not underlined in RNase H active type ASO are deoxyribonucleotides. Nucleotides not underlined in RNase H inactive type ASO are 2'-OMe-nucleotide analogs. ASO was synthesized according to a conventional method. Administration of ASO to cultured iPS cells was performed according to the manufacturer's instructions by using 10 nM ASO together with DharmaFECT1 (Horizon Discovery, Cambridge, UK). FISH using Cy3-labeled DNA/LNA probe that targets UUUCA and Cy5-labeled DNA/LNA probe that targets UUUUA (21 bases for both) was performed 48 hr after administration of ASO to the cultured iPS cells. The results were obtained by observing the same field of view with a fluorescence filter optical system specific to the labeled fluorescence dye Cy3 or Cy5, and DAPI used for counterstaining the cell nuclei, and the number of cell nuclei per fixed area and the number of RNA aggregate-positive nuclei that reacted with the Cy3 probe or Cy5 probe were counted.

### (2) results

Figs. 2A, B and C are graphs showing the results of undifferentiated cells of iPS cells (HPS3905) derived from BAFME patients that were subjected to a lipofection treatment with the addition of ASO of the present Example, undifferentiated cells of HPS3905 (Control Gapmer and Control Mixmer) that were subjected to a lipofection treatment with the addition of negative control ASO instead of the ASO of the present Example, undifferentiated cells (Mock) of HPS3905 that were subjected to a lipofection treatment without the addition of ASO, and undifferentiated cells (Untreated) of HPS3905 that were not subjected to a lipofection treatment. The height of the bar graph of Fig. 2A indicates the percentage of the number of iPS cell nuclei 48 hr after lipofection. The height of the bar graph of Fig. 2B indicates the number of iPS cell nuclei in which RNA aggregates were detected with the Cy3 probe among the iPS cell nuclei 48 hr after lipofection. The height of the bar graph of Fig. 2C indicates the percentage of iPS cell nuclei in which RNA aggregates were detected with the Cy5 probe among the iPS cell nuclei 48 hr after lipofection.

Among the ASOs targeting repetitive sequences, 3009-2M and 3009-3M, 3009-5G and 3009-6G showed a decrease in the percentage of cells in which RNA aggregates are detected (Figs. 2B and C), but the cell numbers (Fig. 2A) did not decrease. All of these ASOs react with RNA containing UUUCA. In contrast, since 3009-1M is an RNase H inactive type ASO that targets UUUUA, the effect was so strong that no cell was found in which RNA aggregates containing UUUUA were detected (Fig. 2C). However, it had almost no effect on the percentage of cells in which RNA aggregates containing UUUCA were detected (Fig. 2B). Even though 3009-4G is a gapmer type ASO that targets UUUUA, the cells in which RNA aggregates containing UUUUA were detected decreased only a little (Fig. 2C), and it had almost no effect on the percentage of cells in which RNA aggregates containing UUUCA were detected (Fig. 2B).

Among the ASOs targeting those other than TTTTA/TTTCA repetitive sequence, SAMD12-M4 and SAMD12-G4 with target sites between 355 and 375 bases 3' downstream from the TTTTA repetitive sequence end site showed a decrease in the percentage of cells in which RNA aggregates are detected (Figs. 2B and C), but the cell numbers did not decrease (Fig. 2A). SAMD12-G1 of the gapmer type ASO with target sites between 71 and 51 bases 5' upstream from the TTTTA repetitive sequence initiation site showed a decrease in the percentage of cells in which RNA aggregates are detected (Figs. 2B and C), but the cell numbers did not decrease (Fig. 2A). In contrast, SAMD12-M1, an RNase H inactive ASO with the same target site, had almost no effect on the percentage of cells in which RNA aggregates were detected (Fig. 2B and Fig. 2C). SAMD12-G2 with a target site between 80 and 100 bases 3' downstream from the TTTTA repetitive sequence end site, and SAMD12-G3 with a target site between 258 and 278 bases 3' downstream from the TTTTA repetitive sequence end site showed a decrease in the number of cells (Fig. 2A). SAMD12-M2 and SAMD12-M3, which have the same target site, did not show a decrease the number of cells (Fig. 2A), and also showed almost no effect on the percentage of cells in which RNA aggregates were detected (Fig. 2B and Fig. 2C) .

From the above results, it was concluded that, among the ASOs tested in this Example, 3009-2M, 3009-3M, 3009-5G, 3009-6G, SAMD12-M4, SAMD12-G4 and SAMD12-G1 can decrease RNA aggregates that are considered to be the cause of BAFME in iPS cells derived from BAFME patients.

### Example 2

### Differentiation of cerebral organoids from patient-derived iPS cells

### (1) material and method

Cerebral organoids were produced by Velasco et al. (Nature 570, 523-527, 2019) after modification as shown in the schematic diagram of Fig. 3A. Briefly, 80% confluent iPS cell colonies were dissociated with Accutase (Innovative Cell Technologies, Funakoshi Co., Ltd.) at 37°C for 5 min, suspended in StemFit (registered trade mark) medium (AK02N, Ajinomoto Co., Inc.) supplemented with 10 µM Y-27632, and the cell number and survival rate were measured. iPS cells were suspended in cortical differentiation medium (Glasgow-MEM containing CDM1, 20% Knockout Serum Replacement (trademark), 0.1 mM non-essential amino acid mixture (NEAA), 1 mM sodium pyruvate, 0.1 mM 2-mercaptoethanol, and 1% penicillin/streptomycin (all Gibco, Thermo Fisher Scientific)) supplemented with 4 µM IWR-1-endo (Calbiochem, Merck Biopharma Co., Ltd.), 5 µM SB431542 (Cayman Chemical, Funakoshi Co., Ltd.), and 20 µM Y-27632, and 9,000-18,000 cells per well were reassembled in ultra-low-cell-adhesion V-bottom 96-well plates (Sumitomo Bakelite Co., Ltd.). From day 6 to day 18, cell aggregates were cultured in CDM1 medium supplemented with 3 µM IWRI and 5 µM SB431542. From day 18, floating aggregates were cultured with rotary shaking at a speed of 60 rpm in CDM2 medium containing DMEM/F12 medium, 2 mM GlutaMAX (trademark), 1% N2 Supplement, 1% Chemically Defined Lipid Concentrate, 0.25 µg/mL amphotericin B, and 1% penicillin/streptomycin (all Gibco, Thermo Fisher Scientific) placed in a 60 mm culture dish (Corning Incorporated). From day 35, the organoids were transferred to CDM3 medium consisting of CDM2 supplemented with 10% fetal bovine serum (FBS, Biosera, FUJIFILM Wako Pure Chemical Corporation), 5 µg/mL heparan (Sigma Ltd., Merck Biopharma Co., Ltd.), and 1% Matrigel (Growth Factor Reduced, Corning Incorporated). From day 70, the organoids were cultured in CDM4 consisting of CDM3 supplemented with B27 Supplement (Gibco, Thermo Fisher Scientific) and 2% Matrigel. ASO administration was performed by transferring cerebral organoids to CDM4 medium without Matrigel but containing 500 nM ASO.

Cerebrum organoids were fixed with 4% paraformaldehyde for 30 min at room temperature, washed three times with PBS for 10 min, immersed in 30% sucrose solution overnight, embedded in OCT compound, and quickly frozen in liquid nitrogen. 12 µm-thick frozen sections were produced with a cryostat at -18°C to -20°C. For FISH, permeabilization was performed in 0.5% Triton-X100/PBS for 30 min at room temperature, and RNA aggregates were detected by the FISH method in the same manner as in Example 1. For multiple immunostaining, anti-MAP2 antibody (Millipore, Merck Co., Ltd.), anti-PAX6 antibody (Biolegend, Tommy Digital Biology Co., Ltd.), and anti-SOX2 antibody (eBioscience, Thermo Fisher Scientific Co., Ltd.) were used as the primary antibodies. Blocking was performed at room temperature for 2 hr in Blocking One Histo (Nacalai Tesque, Inc.). Sections were incubated overnight at 4°C with primary antibody diluted in PBS containing Blocking One Histo and 0.1% Triton-X100. After washing four times in 0.1% Triton-X100/PBS, the sections were incubated with the secondary antibody for 2 hr at room temperature, and washed four times with 0.1% Triton-X100/PBS. They were mounted using ProLong (trademark) Gold Antifade Mountant (Thermo Fisher Scientific Co., Ltd.). Image data were acquired using a fluorescence confocal microscope system (A1, Nikon Corporation) and analyzed using NIS-Element AR Analysis software (version 5.11.01, 64-bit, Nikon Corporation).

Fig. 3B shows multiple immunostaining fluorescence microscopic images of cerebral organoid sections obtained by culturing iPS cell line HPS3905 derived from BAFME patients for 70 days. The scale bar is 50 µm. MAP2, PAX6, and SOX2 are markers of neuron, neural progenitor, and glial cell, respectively. Neural tube-like structures and neural cells were observed in Fig. 3B.

Fig. 3C shows fluorescence microscopic images of FISH using Cy3 probe and DAPI counterstaining of cerebral organoid sections obtained by culturing iPS cell line HPS3905 derived from BAFME patients for 70 days. The scale bar is 10 µm. In Fig. 3C, RNA aggregates were observed in the nuclei.

Fig. 3D is a graph showing the percentage of cell nuclei in which RNA aggregates were detected with the Cy3 probe in the cell nuclei of cerebral organoid sections obtained by culturing iPS cell line HPS3905 (BAFME1) derived from BAFME patients and iPS cell line 201B7 (HC) derived from healthy subjects for 70 days. From Fig. 3D, unlike cell nuclei derived from healthy subjects, RNA aggregates were detected in 40% of cell nuclei derived from BAFME patients also in cerebral organoids, like undifferentiated iPS cells.

Fig. 3E is a graph showing the effect of the ASO of the present Example (ASO-4M, same as 3009-2M) or negative control ASO (Control ASO, same as Control Mixmer) on RNA aggregate formation in cerebral organoids prepared from iPS cells derived from BAFME patients. It shows the percentage of cell nuclei in which RNA aggregates were detected with the Cy3 probe in the cell nuclei of sections obtained by culturing iPS cell line HPS3905 derived from BAFME patients for 5 months to give cerebral organoids, administering thereto ASO-4M or Control ASO for 1 week by lipofection, and fixing. From Fig. 3E, administration of ASO-4M, which is a mixmer ASO targeting UUUCA repeats, suppressed RNA aggregate formation in the nuclei of cells derived from BAFME patients. This suggests that administration of the ASO of the present invention to BAFME patients may exhibit therapeutic efficacy by suppressing formation of RNA aggregates.

This application is based on a patent application No. 2021-009903 filed in Japan (filing date: January 25, 2021), the contents of which are incorporated in full herein.

### [Industrial Applicability]

The ASO of the present invention is useful as a preventive or therapeutic agent for BAFME.

## Claims

1. A preventive or therapeutic agent for benign adult familial myoclonus epilepsy (BAFME), comprising an antisense oligonucleotide comprising at least one of the base sequences of SEQ ID NOs: 1 to 4.

2. The agent according to claim 1, wherein an oligonucleotide consisting of at least one of the base sequences of SEQ ID NOs: 1 to 4 of the antisense oligonucleotide comprises at least one RNase H inactive type nucleotide analog.

3. The agent according to claim 2, wherein the RNase H inactive type nucleotide analog is at least one kind of nucleotide analog selected from the group consisting of a nucleotide analog in which the 2'-position of ribose is modified, and a nucleotide analog modified by bridging between the 2'-position and the 4'-position of ribose.

4. The agent according to claim 3, wherein the nucleotide analog in which the 2'-position of ribose is modified is at least one kind of nucleotide analog selected from the group consisting of a 2'-OMe-nucleotide analog, and an MOE-nucleotide analog.

5. The agent according to claim 3 or 4, wherein the nucleotide analog modified by bridging between the 2'-position and the 4'-position of ribose is at least one kind of nucleotide analog selected from the group consisting of β-D-oxy-L-LNA, β-D-ENA, and R type cEt.

6. The agent according to any one of claims 1 to 5, wherein at least two adjacent nucleotides of the oligonucleotide consisting of a base sequence of any of SEQ ID NOs: 1 to 4 of the antisense oligonucleotide are linked by a modified internucleoside bond.

7. The agent according to claim 6, wherein all adjacent nucleotides of the oligonucleotide consisting of a base sequence of any of SEQ ID NOs: 1 to 4 of the antisense oligonucleotide are linked by a modified internucleoside bond.

8. The agent according to claim 6 or 7, wherein the modified internucleoside bond is at least one kind of bond selected from the group consisting of a phosphorothioate bond, a phosphorodithioate bond, and a boranophosphate bond.

9. The agent according to any one of claims 1 to 8, wherein the antisense oligonucleotide is RNase H active type.

10. The agent according to claim 9, wherein the 1st to 5th nucleotides from the 5'-terminal and the 16th to 20th nucleotides from the 5'-terminal of the oligonucleotide consisting of a base sequence of any of SEQ ID NOs: 1 to 4 are RNase H inactive type nucleotide analogs, and the 6th to 15th nucleotides from the 5'-terminal of the oligonucleotide therein are RNase H active type nucleotides.

11. The agent according to any one of claims 1 to 8, wherein the antisense oligonucleotide comprising the oligonucleotide consisting of a base sequence of SEQ ID NO: 1, 2, or 3 is RNase H inactive type.

12. The agent according to claim 11, wherein all nucleotides including thymine of the oligonucleotide consisting of the base sequence of SEQ ID NO: 1 or 2 are nucleotide analogs modified by bridging between the 2'-position and the 4'-position of ribose,
nucleotides comprising the 5th, 10th, 15th, and 20th adenines from the 5'-terminal of the oligonucleotide consisting of the base sequence of SEQ ID NO: 1 or 2 are nucleotide analogs modified by bridging between the 2'-position and the 4'-position of ribose, and
other nucleotides are all nucleotide analogs in which the 2'-position of ribose is modified.

13. The agent according to claim 12, wherein all the nucleotide analogs modified by bridging between the 2'-position and the 4'-position of ribose are β-D-ENA, and all the nucleotide analogs in which the 2'-position of ribose is modified are 2'-OMe-nucleotide analogs.

14. The agent according to claim 11, wherein all nucleotides including cytosine and thymine of the oligonucleotide consisting of the base sequence of SEQ ID NO: 3 are nucleotide analogs modified by bridging between the 2'-position and the 4'-position of ribose, and
all other nucleotides are nucleotide analogs in which the 2'-position of ribose is modified.

15. The agent according to claim 14, wherein all the nucleotide analogs modified by bridging between the 2'-position and the 4'-position of ribose are β-D-ENA, and all the nucleotide analogs in which the 2'-position of ribose is modified are 2'-OMe-nucleotide analogs.
